# EUROPEAN PATENT SPECIFICATION

(11) **EP 4 435 408 B1**
(45) Date of publication and mention of the grant of the patent: **22.01.2025**
(21) Application number: 23162941.1
(22) Date of filing: 20.03.2023
(51) Int. Cl.: G01N 21/31, G01N 21/64, G01N 33/18, G01J 3/02, G01J 3/18, G01J 3/44, G01N 21/53, G01N 21/47, G01N 21/63

(54) **AN AUTOMATED OPTICAL SPECTROSCOPY DEVICE FOR WATER ANALYSIS**
AUTOMATISCHE OPTISCHE SPEKTROSKOPIEVORRICHTUNG ZUR WASSERANALYSE
DISPOSITIF DE SPECTROSCOPIE OPTIQUE AUTOMATISÉ POUR ANALYSE D'EAU

(43) Date of publication of application: 25.09.2024
(73) Proprietor: Latvijas Universitates Cietvielu fizikas instituts, 1063 Riga (LV)
(72) Inventor: Zolotarjovs, Aleksejs, LV-1045 Riga (LV); Tunens, Gatis, LV-1029 Riga (LV); Einbergs, Ernests, LV-1057 Riga (LV); Vilks, Karlis, LV-1048 Riga (LV); Fomins, Sergejs, LV-2167 Marupe (LV)
(74) Representative: Fortuna, Jevgenijs

(56) References cited:
- CN-A- 104 730 054
- HANS BARTH ET AL: "In-situ analysis of water quality: spectral attenuation and fluorescence", PROCEEDINGS OF SPIE, vol. 3107, 23 May 1997 (1997-05-23), US, XP055295681, ISBN: 978-1-5106-1533-5, DOI: 10.1117/12.274734

## Description

### Technical Field

The invention relates to devices for performing *in-situ* monitoring of water by means of spectroscopy.

### Background Art

In a rapidly growing industry of fish/marine farming, the need for sensor systems capable to analyze water quality is steadily increasing. Reliable and rapid sensor data is needed to properly implement data-driven farming approaches in automated and scalable scenarios.

There are known different approaches to measure some parameters describing water quality: electrical (using two submerged electrodes and an electrical device configured to analyze signal), chemical (adding other chemical components and observing reactions), physical deformation (heating/boiling or cooling of water and analyzing the results in form of vapor, residual water etc), and optical (using more or less complex optical devices to measure such water optical parameters, as absorbance, fluorescence, phosphorescence and other). A combination of several methods may be used to characterize the same parameter.

With respect to the sampling, the measurements can be performed in invasive, intrusive, or *in-situ* configuration, when sensor is inserted inside the water and operated within. Alternatively, a sample can be taken in a vial to the lab where it is analyzed with different methods. The second method is much more popular for now as it is the cheapest and most precise. However, sample removal approach delays the results and cannot be used in a data-driven farming due to the low sampling rate - typically, once in couple of days.

For invasive measurements, some sensor types are more suitable than others, with one of the better solutions being optical sensors. Naturally, there are a lot of existing optical sensors for measurement of water parameters, like chlorophyll, cyanobacteria concentration. However, they operate in a single wavelength configuration or use a fixed configuration of some type of optical system involving VIS photometers.

There is known an underwater imaging spectrometer in-situ measurement system (CN213600570U), comprising a frame; a sensor assembly, arranged on one side of the frame; the sensor assembly comprising irradiance sensor and radiance sensor; the device further comprising a camera assembly, wherein a camera is attached to the irradiance sensor or the radiance sensor and arranged on the frame; the irradiance sensor, the radiance sensor and the camera are all arranged on the same horizontal plane.

There are known controllable buoys and networked buoy systems (US9563203B2) comprising floating buoys with sensors and energy harvesting capabilities. The buoys are configured to control their buoyancy and motion, and to organize communication in a distributed fashion. The buoys have tethered underwater vehicles with a smart spooling system that allows the vehicles to dive deep underwater while remaining in communication and connection with the buoys.

There is known a fiber buoy for online monitoring of water quality (CN104807759A) comprising a solar cell panel, a power supply module, a support, a navigation mark lamp, an antenna, a light source, a fiber coupler, a connecting fiber, a fiber sensing head, a reflection end surface, an optical switch, a spectrometer module and a GPRS module. The solar cell panel is connected with the battery module, the solar cell panel and the battery module are arranged on the support, and the navigation mark lamp and the antenna are arranged at the top of the support. The output end of the light source is connected with the fiber coupler, the fiber coupler is connected with one end of the sensing head, and the other end of the sensing head plates a high reflection film on the end surface of the fiber to form the reflection end surface.

There is known a rotating flexible filter spectrometer (EP0655129B1) using flexible variable filters, which are mounted within the circumference of a cylinder. The design allows to create a simple focusing system. The spectrometer is configurable to scan a variety of ranges by changing the filter cylinders, or by adding additional filter cylinders.

There is known an integrated probe type photoelectric water-quality multiparameter online measuring system (CN104730054A), comprising a control and data acquiring unit and an optical system unit. The optical system unit comprises a composite light source with two light sources: one light source is an LED array composed of LED light sources emitting light at different visible wavelengths, and another light source is composed of ultraviolet LED light sources and near-infrared laser diode light sources. The optical system unit further comprises a photodetector for detecting fluorescent or scattered light from a sample. The light from the sample is collected by a lens and passed through one of two bandpass filters or a blank in a rotatable filter wheel arranged between the lens and the photodetector. The computer controls the light source to emit different wavelengths of light to irradiate water body to be detected by the control and data acquiring unit and triggers substances of the water body to be detected to emit fluorescent and scattered light signals.

The main disadvantage of the known solutions is insufficient variability, which may be impacted by other pollutants that are fluorescent in the same spectral range; limitation of the system not allowing to distinguish between overlapping peaks from different water components; loss of information due to the excitation diodes being in the range of detection of the detector; insufficient flexibility of the system not allowing to use the system for measuring wide variety of parameters, or relative complexity of construction; as well as inability to use the same detection system for both absorption and phosphorescence measurements

### Summary of the Invention

The goal of the invention is to overcome the drawbacks of the prior art and to provide a simple and effective optical spectroscopy device for water analysis, which would allow to obtain significantly large set of data specific to common pollutants and components of water.

The set goal is achieved by providing an optical spectroscopy device for water analysis as defined in claim 1, a system comprising the optical spectroscopy device as defined in claim 6, and a floating construction comprising the system as defined in claim 9. The optical spectroscopy device comprises: an optical microspectrometer; and a waterproof casing designed to withstand underwater environment; the casing having at least one portion, which is transparent to the wavelengths of light being measured; the microspectrometer comprising a light source; a detector configured to detect and measure a spectral signal emitted, transmitted, or reflected by a sample; optical components comprising optical lenses and optical filters; and a means for controllable positioning of the optical lenses and the optical filters in front of the microspectrometer's detector; wherein the optical filters comprise a 390-410 nm high pass filter, a 490-510 nm high pass filter, and a 590-610 nm high pass filter; wherein the light source is a light source or a set of light sources, designed to emit light in the ranges 360-380 nm, 440-460 nm, and 570-590 nm; wherein the microspectrometer is accommodated within the casing, so that the detector is positioned behind the transparent portion of the casing to be capable to be exposed to the spectral signal being measured.

The means for controllable positioning of the optical filters may comprise a motorized optical filter wheel or plate with a series of filter slots on the filter wheel or plate.

The optical spectroscopy device for water analysis is configured to be connected to a power source, which may be a rechargeable battery. The rechargeable battery may be accommodated in the waterproof casing together with the optical microspectrometer, or be located outside of the casing of the optical microspectrometer.

The optical components of the device may further comprise at least one mirror for directing the light to the microspectrometer. Also, the device may further comprise a monochromator, having a grating or prism designed to disperse the light into its component wavelengths, and a slit designed to allow only a specific wavelength range to pass through; the microspectrometer is further designed to direct the monochromatic light onto the sample and measure by the microspectrometer's detector the light scattered or emitted by the sample.

According to the preferred embodiment, the waterproof casing is further designed to accommodate a wireless communication module that allows the submersible optical spectroscopy device for water analysis to communicate wirelessly with a remote device; wherein the means for controllable positioning of the optical filters is configured to be remotely operable using a wireless communication system. According to yet preferred embodiment, the sensor further comprises one or more sensors or feedback mechanisms, configured to provide information about the position and status of the optical filters, and transmit that information back to the remote device, to enable real-time monitoring and control.

The invention also provides a system comprising the optical spectroscopy device and a part configured to be located fully or partially above the surface of the water, the part provided with a detector and configured to measure ambient illumination of the water by means of the detector, wherein the part and the spectroscopy device are electrically connected. The invention also provides a floating construction comprising the system. The floating construction (which may be a floating fish or bivalve farm, a buoy, a watercraft, a platform, a floating dock, or a pontoon) further comprises a processing unit configured to receive and interpret commands from the remote device, and execute them by sending a signal to the means for controllable positioning of the optical lenses and the optical filters to adjust the position of the optical lenses and the optical filters; a communication system configured to allow the processing unit to receive data from the submersible optical spectroscopy device for water analysis, and transmit that data to a remote location for further processing or analysis. It may optionally comprise also a memory adapted to allow the processing unit to store data collected by the submersible optical spectroscopy device for water analysis, for later retrieval and analysis.

### Description of Drawings

Fig. 1 - a general representation of an embodiment of the submersible optical spectroscopy device for water analysis assembled in a waterproof casing;
Fig. 2 -a general representation of an embodiment of a disassembled submersible optical spectroscopy device for water analysis (without a casing), provided with an integrated filter and LED system;
Fig. 3 - an embodiment, comprising two connected devices: a submersible and above water optical spectroscopy device for water analysis.

### Detailed Description of the Invention

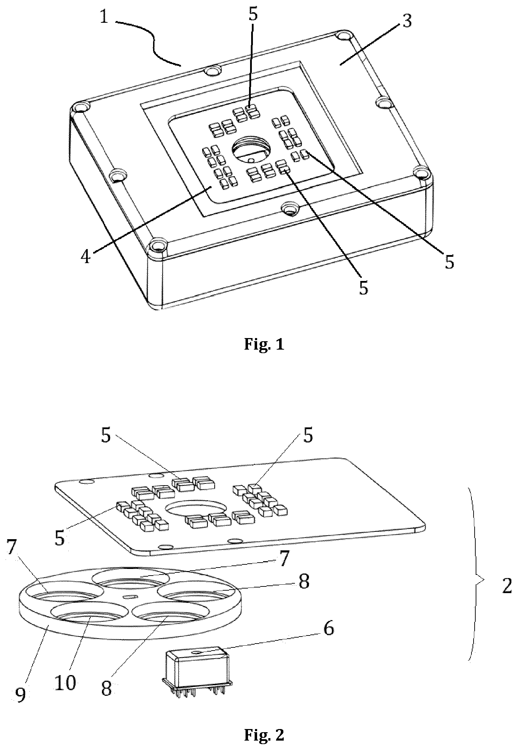

Figures 1 and 2 show the submersible optical spectroscopy device 1 for water analysis, which comprises: an optical microspectrometer 2; and a waterproof casing 3 designed to withstand underwater environment and accommodate the microspectrometer 2. The casing 3 having at least one portion 4, which is transparent to the wavelengths of light being measured. The microspectrometer 2 comprises a light source 5; a detector 6 configured to detect and measure the spectral signal emitted, transmitted, or reflected by a sample; optical components comprising optical lenses 7 and optical filters 8; and a means 9 for controllable positioning of the optical lenses 7 and optical filters 8 in front of microspectrometer's detector 6. The optical filters 8 comprise a 390-410 nm high pass filter, a 490-510 nm high pass filter, and a 590-610 nm high pass filter. The light source 5 is a light source or a set of light sources, designed to emit light in the ranges 360-380 nm, 440-460 nm, and 570-590 nm, providing the illumination needed to excite the sample and generate a spectral signal. The microspectrometer 2 is designed to be accommodated within the casing 3, so that the detector 6 is positioned behind the transparent portion 4 of the casing 3 to be capable to be exposed to the light signal being measured. According to the preferred embodiment the means 9 for controllable positioning of the optical lenses 7 and optical filters 8 further comprise an empty slot 10 for absorbance measurements.

The means 9 for controllable positioning of the optical lenses and filters may comprise a motorized optical filter wheel or a plate with a series of filter slots on the filter wheel or plate. The motorized optical filter wheel or a plate is designed to be controlled by a processing unit, which sends commands to the motor to move the wheel or the plate to the desired position. The speed and precision of the motor can be adjusted to ensure accurate and repeatable positioning of the lenses 7 and the filters 8.

The device 1 is configured to be connected to a power source, which may be a rechargeable battery. The rechargeable battery may be accommodated in the waterproof casing 3 together with the optical microspectrometer 2, or be located outside of the casing 3 of the optical microspectrometer 2.

The optical components of the device 1 may further comprise at least one mirror for directing the light to the detector 6. Also, the device may further comprise a monochromator, having a grating or prism designed to disperse the light into its component wavelengths, and a slit designed to allow only a specific wavelength range to pass through. The microspectrometer 2 may be further designed to direct the monochromatic light onto the sample and measure by the microspectrometer's detector the light scattered or emitted by the sample.

According to the preferred embodiment, the waterproof casing 3 further designed to accommodate a wireless communication module that allows the device 1 to communicate wirelessly with a remote device; wherein the means 9 for controllable positioning of the optical lenses and filters is configured to be remotely operable using a wireless communication system. According to yet preferred embodiment, the device 1 further comprises one or more sensors or feedback mechanisms, configured to provide information about the position and status of the optical lenses 7 and filters 8, and transmit that information back to the remote device, to enable real-time monitoring and control.

The invention also provides for a floating construction (which may be a floating fish or bivalve farm, a buoy, a watercraft, a platform, a floating dock, or a pontoon), comprising: the submersible optical water quality device 1, attached to the floating construction below the surface of water; a processing unit configured to receive and interpret commands from the remote device, and execute them by sending a signal to the means 9 for controllable positioning of the optical lenses and filters to adjust the position of the optical lenses and filters; a communication system configured to allow the processing unit to receive data from the submersible optical spectroscopy device 1 for water analysis, and transmit that data to a remote location for further processing or analysis. It may optionally comprise also a memory adapted to allow the processing unit to store data collected by the device 1, for later retrieval and analysis.

The device allows to effectively determine relative concentrations of chlorophyll and phycocyanin in the measurement environment. It allows to measure different types of excitations, such as the primary excitation to get a base measurement of any photoluminescent compounds found in water, including any dissolved organic matter (DOM) and chlorophyll; the chlorophyll absorption maximum, allowing for direct detection of chlorophyll and the phycocyanin. Each excitation source can be combined with one of the device's optical filters 7 to remove excitation light from the detectable signal. As the system includes a spectrometer, absorption can be measured as well - during daylight by taking a sunlight reference spectrum with the additional spectrometer above the surface of water or by a calibrated light source during night time. This allows not only for the detection of non-photoluminescent compounds, but also to detect chlorophyll and other molecules that are absorbent within the visible light spectral range. The empty space in the means 9 allows to perform these measurements without direct excitation from the light source 5. In case of background light, such as a sunlight, floodlight, or a calibration source can be used.

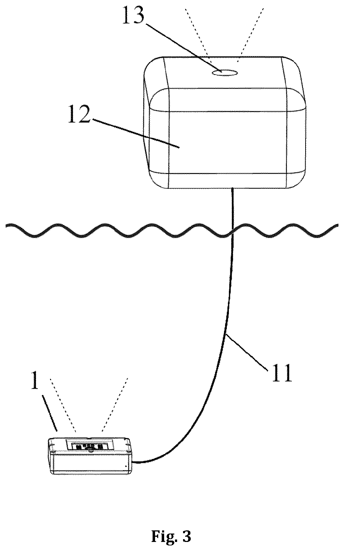

According to an embodiment, the spectroscopy device 1 for water analysis may comprise a submersible part and a part 12 located fully or partially above the surface of water (Fig. 3). The above water part 12 can be used for the collection of reference spectrum of the sun and be connected to the same data processing unit as the submersible spectroscopy device 1 for water analysis. The above water part 12 can have the same elements as the underwater one, but without filters and LEDs and be configured to monitor ambient illumination above the water through a detector 13 simultaneously with underwater spectral measurement by the submersible part of the device 1. The submersible and above water parts of the device can be electrically connected by a cable 11.

To perform measurements, the 390-410 nm high pass filter is used in combination with 360-380 nm LED diode to evaluate overall shape of DOM maximum present in any liquid containing organics. In addition, presence of oils and oil products can be detected with this combination. The 490-510 nm high pass filter is used in combination with 360-380 nm LED diode to exclude most intense part of DOM and increase the sensitivity of its yellow part. The 590-610 nm high pass filter is used in combination with 360-380 nm LED diode to distinguish chlorophyll peak in the red region, as well as see the presence of any organic pigments that are participating in photosynthesis processes in plants. The 440-460 nm LED diode is used in two correlative measurements - with 590-610 nm high pass filter and 490-510 nm high pass filter - former is used to detect only chlorophyll maximum and the latter is used to see the DOM peak impact on chlorophyll signal. The 590-610 nm high pass filter is used in combination with 570-590 nm LED diode to detect phycocyanin present in cyanobacteria. The spectrometer can be used without filters and LEDs to perform an absorption measurement using the light from the atmosphere. The spectrum can be taken simultaneously on two spectrometers - one inside the submerged part, and one above the surface of water. By signal filtering and subtraction, it is possible to measure the absorption only by the water, thus detecting presence of compounds such as pheophorbide and pheophytin.

According to an ambodiment, the spectroscopy device 1 for water analysis comprises the detector 6, measuring the spectral signal under water and the detector 13 measuring the spectral signal above the water; a processing unit operatively connected to the detectors 6 and 13, wherein the processing unit is configured to: (i) control the detector 6 and the detector 13 to measure spectra simultaneously to exclude the effects of any fast-changing light conditions; (ii) determine an optimal exposure time for the detector 13 based on the measured above water light spectra; (iii) adjust the exposure time of the detector 6 to match the determined optimal exposure time of the detector 13; (iv) obtain intensity values for each pixel of the measured spectra from the detector 6 and the detector 13; (v) apply correction coefficients C₁ₙ and C₂ₙ to the intensity values of each pixel of the measured spectra, wherein the correction coefficients account for spectral sensitivity, light intensity sensitivity, readout electronics non-linearity, and, optionally, any other non-linear device-specific effects; (vi) calculate a resulting absorption spectrum Aₙ for each pixel (n) using the formula Aₙ = C₁ₙ * Bₙ - C₂ₙ * Dₙ, where Bₙ is the intensity registered on the above water detector and Dₙ is the intensity registered on the underwater detector; and (vii) correlate the resulting absorption spectra to analyze water properties.

The claimed invention ensures data precision even in conditions where many overlapping signals are present. The use of phosphorescence and absorption datasets also increase the flexibility of the setup for measurement of wide variety of components.

The data obtained can then be used to rapidly address public health hazards, locate failures in filtration systems, react on sudden changes in quality due to the oil spills and make predictions on upcoming phytoplankton blooms, adjust the amount of food provided to fish stock.

## Claims

1. A spectroscopy device (1) for water analysis comprising: an optical microspectrometer (2); and a waterproof casing (3) designed to withstand underwater environment; the casing (3) having at least one portion (4), which is transparent to the wavelengths of light being measured; the microspectrometer (2) comprising a light source (5); a detector (6) configured to detect and measure a spectral signal emitted, transmitted, or reflected by a sample; optical components comprising optical lenses (7) and optical filters (8); and a means (9) for controllable positioning of the optical lenses (7) and the optical filters (8) in front of the microspectrometer's detector (6); wherein the optical filters (8)comprise a 390-410 nm high pass filter, a 490-510 nm high pass filter, and a 590-610 nm high pass filter; wherein the light source (5) is a light source or a set of light sources, designed to emit light in the ranges 360-380 nm, 440-460 nm, and 570-590 nm; wherein the microspectrometer (2) is designed to be accommodated within the casing (3), so that the detector (6) is positioned behind the transparent portion (4) of the casing (3) to be capable to be exposed to the spectral signal being measured.

2. The device (1) of claim 1, wherein the means (9) for controllable positioning of the optical lenses and optical filters comprises a motorized optical filter wheel or plate with a series of filter slots on the filter wheel or plate.

3. The device (1) of any preceding claims, further comprising a power source, wherein the power source comprises a rechargeable battery.

4. The device (1) of claim 1, wherein the optical components further comprise at least one mirror for directing the light to the microspectrometer.

5. The device (1) of claim 1, wherein the microspectrometer (2) further comprises a monochromator, comprising a grating or prism designed to disperse the light into its component wavelengths, and a slit designed to allow only a specific wavelength range to pass through; and wherein the microspectrometer (2) is further designed to direct the monochromatic light onto the sample and measure by the microspectrometer's detector (6) the light scattered or emitted by the sample.

6. A system, comprising the spectroscopy device (1) of claim 1 and a part (12) configured to be located fully or partially above the surface of water, the part (12) provided with a detector (13); the part (12) configured to measure ambient illumination above the water by means of the detector (13); wherein the part (12) and the spectroscopy device (1) are electrically connected.

7. The system of claim 6, wherein the waterproof casing (3) further designed to accommodate a wireless communication module that allows the device (1) to communicate wirelessly with a remote device; wherein the means (9) for controllable positioning of the optical lenses and optical filters is configured to be remotely operable using a wireless communication system.

8. The system of claim 7, further comprising one or more sensors or feedback mechanisms, configured to provide information about the position and status of the optical lenses (7) and the optical filters (8), and transmit that information back to the remote device, to enable real-time monitoring and control.

9. A floating construction, comprising:
the system of claim 7 or claim 8;
a processing unit configured to receive and interpret commands from the remote device, and execute them by sending a signal to the means for controllable positioning of the optical lenses (7) and the optical filters (8) to adjust the position of the optical lenses (7) and the optical filters (8);
a communication system configured to allow the processing unit to receive data from the device (1), and transmit that data to a remote location for further processing or analysis;
optionally, a memory adapted to allow the processing unit to store data collected by the device (1), for later retrieval and analysis.

## Patentansprüche

1. Ein spektroskopisches Gerät (1) zur Wasseranalyse, der sich aus folgenden Bestandeilen zusammensetzt: einem optischen Mikrospektrometer (2), einem wasserdichten Gehäuse (3), das so beschaffen ist, dass es einer Unterwasserumgebung standhält, wobei das Gehäuse (3) mindestens einen Abschnitt (4) aufweist, der für die Wellenlängen des zu messenden Lichts transparent ist; das Mikrospektrometer (2) enthält eine Lichtquelle (5), einen Detektor (6), der so ausgelegt ist, dass er ein von einer Probe ausgestrahltes, durchgelassenes oder reflektiertes Spektralsignal erfasst und misst; optische Komponenten, die optische Linsen (7) und optische Filter (8) enthalten; und ein Steuergerät (9) zur Positionierung der optischen Linsen (7) und der optischen Filter (8) vor dem Detektor (6) des Mikrospektrometers. Die optischen Filter (8) enthalten einen Hochpassfilter mit 390-410 nm, einen Hochpassfilter mit 490-510 nm und einen Hochpassfilter mit 590-610 nm. Die Lichtquelle (5) ist eine Lichtquelle oder ein Satz von Lichtquellen, die dafür ausgelegt sind, Licht in den Bereichen 360-380 nm, 440-460 nm und 570-590 nm auszustrahlen. Das Mikrospektrometer (2) ist so konzipiert, dass es in dem Gehäuse (3) untergebracht werden kann, so dass der Detektor (6) hinter dem transparenten Teil (4) des Gehäuses (3) angeordnet ist, um dem zu messenden Spektralsignal ausgesetzt werden zu können.

2. Gerät (1) gemäß Patentanspruch 1, wobei das Steuergerät (9) zur Positionierung der optischen Linsen und der optischen Filter ein angetriebenes optisches Filterrad oder eine optische Filterplatte mit einer Reihe von Filterschlitzen auf dem Filterrad oder der Filterplatte enthalten.

3. Gerät (1) gemäß einem der vorhergehenden Patentansprüche umfasst ferner eine Energiequelle, wobei die Energiequelle einen wiederaufladbaren Akku enthält.

4. Gerät (1) gemäß Patentanspruch 1, wobei die optischen Komponenten ferner mindestens einen Spiegel umfassen, um das Licht auf das Mikrospektrometer zu lenken.

5. Gerät (1) gemäß Patentanspruch 1, wobei das Mikrospektrometer (2) ferner einen Monochromator mit einem Gitter oder Prisma enthält, der dafür ausgelegt ist, das Licht in seine einzelnen Wellenlängen aufzuteilen, sowie einen Spalt, der so gestaltet ist, dass er nur einen bestimmten Wellenlängenbereich durchlässt. Das Mikrospektrometer (2) ist ferner so beschaffen, dass es das monochromatische Licht auf die Probe richtet und mit dem Detektor (6) des Mikrospektrometers das von der Probe gestreute oder ausgestrahlte Licht misst.

6. Ein System, das aus dem spektroskopischen Gerät (1) gemäß Anspruch 1 und einem Teil (12) besteht, das so konfiguriert ist, dass es sich ganz oder teilweise über der Wasseroberfläche befindet. Das Teil (12) ist dabei mit einem Detektor (13) ausgestattet und so eingestellt, dass es die Umgebungsbeleuchtung über dem Wasser mit Hilfe des Detektors (13) misst. Das Teil (12) und das spektroskopische Gerät (1) sind elektrisch miteinander verbunden.

7. System gemäß Patentanspruch 6, wobei das wasserdichte Gehäuse (3) ferner so gestaltet ist, dass es ein drahtloses Kommunikationsmodul aufnimmt, das es dem Gerät (1) ermöglicht, drahtlos mit einem entfernten anderen Gerät zu kommunizieren. Das Steuergerät (9) zur Positionierung der optischen Linsen und der optischen Filter ist dabei so eingestellt, dass es unter Verwendung eines drahtlosen Kommunikationssystems aus der Ferne bedient werden kann.

8. System gemäß Patentanspruch 7 bestehend des Weiteren aus einem oder mehreren Sensoren oder Rückkopplungsmechanismen, die dafür vorgesehen sind, Informationen über die Position und den Zustand der optischen Linsen (7) und der optischen Filter (8) zu liefern und diese Informationen an das Ferngerät zurücksenden, um eine Überwachung und Steuerung in Echtzeit zu ermöglichen.

9. Schwimmende Konstruktion, bestehend aus: dem System gemäß Patentanspruch 7 oder 8; einer Verarbeitungseinheit, die so konfiguriert ist, dass sie Befehle vom Ferngerät empfängt, interpretiert und ausführt, indem sie ein Signal an das Steuergerät zur Positionierung der optischen Linsen (7) und der optischen Filter (8) sendet, um diese einzustellen; einem Kommunikationssystem, das so konfiguriert ist, dass es der Verarbeitungseinheit ermöglicht, Daten vom Gerät (1) zu empfangen und diese Daten zur weiteren Verarbeitung oder Analyse per Fernübertragung zu übermitteln; einem optionalen Speicher, der so angepasst ist, dass er es der Verarbeitungseinheit ermöglicht, vom Gerät (1) gesammelte Daten zu speichern, um sie später abzurufen und zu analysieren.

## Revendications

1. Dispositif de spectroscopie (1) destiné à une analyse d'eau comprenant : un microspectromètre (2) optique ; et un boîtier étanche (3) conçu pour supporter un environnement subaquatique ; le boîtier (3) ayant au moins une portion (4), qui est transparente aux longueurs d'onde de lumière étant mesurées ; le microspectromètre (2) comprenant une source de lumière (5) ; un détecteur (6) configuré pour détecter et mesurer un signal spectral émis, transmis ou réfléchi par un échantillon ; des composants optiques comprenant des lentilles optiques (7) et des filtres optiques (8) ; et un moyen (9) permettant un positionnement commandable des lentilles optiques (7) et des filtres optiques (8) devant le détecteur (6) du microspectromètre ; dans lequel les filtres optiques (8) comprennent un filtre passe-haut de 390 à 410 nm, un filtre passe-haut de 490 à 510 nm et un filtre passe-haut de 590 à 610 nm ; dans lequel la source de lumière (5) est une source de lumière ou un ensemble de sources de lumière, conçu pour émettre de la lumière dans les plages 360 à 380 nm, 440 à 460 nm et 570 à 590 nm ; dans lequel le microspectromètre (2) est conçu pour être logé au sein du boîtier (3), de sorte que le détecteur (6) est positionné derrière la portion transparente (4) du boîtier (3) pour pouvoir être exposé au signal spectral étant mesuré.

2. Dispositif (1) selon la revendication 1, dans lequel le moyen (9) permettant un positionnement commandable des lentilles optiques et des filtres optiques comprend une roue ou plaque filtrante optique motorisée avec une série de fentes filtrantes sur la roue ou plaque filtrante.

3. Dispositif (1) selon de quelconques revendications précédentes, comprenant en outre une source de puissance, dans lequel la source de puissance comprend une batterie rechargeable.

4. Dispositif (1) selon la revendication 1, dans lequel les composants optiques comprennent en outre au moins un miroir permettant de diriger la lumière vers le microspectromètre.

5. Dispositif (1) selon la revendication 1, dans lequel le microspectromètre (2) comprend en outre un monochromateur, comprenant un réseau ou prisme conçu pour disperser la lumière en ses longueurs d'onde composantes, et une fente conçue pour ne laisser passer qu'une plage de longueur d'onde spécifique ; et dans lequel le microspectromètre (2) est en outre conçu pour diriger la lumière monochromatique sur l'échantillon et mesurer par le détecteur (6) du microspectromètre la lumière diffusée ou émise par l'échantillon.

6. Système, comprenant le dispositif de spectroscopie (1) selon la revendication 1 et une partie (12) configurée pour être localisée complètement ou partiellement au-dessus de la surface de l'eau, la partie (12) étant pourvue d'un détecteur (13) ; la partie (12) étant configurée pour mesurer un éclairage ambiant au-dessus de l'eau au moyen du détecteur (13) ; dans lequel la partie (12) et le dispositif de spectroscopie (1) sont connectés électriquement.

7. Système selon la revendication 6, dans lequel le boîtier étanche (3) est en outre conçu pour recevoir un module de communication sans fil qui permet au dispositif (1) de communiquer sans fil avec un dispositif distant ; dans lequel le moyen (9) permettant un positionnement commandable des lentilles optiques et des filtres optiques est configuré pour pouvoir fonctionner à distance à l'aide d'un système de communication sans fil.

8. Système selon la revendication 7, comprenant en outre un ou plusieurs capteurs ou mécanismes de rétroaction, configurés pour fournir des informations concernant la position et l'état des lentilles optiques (7) et des filtres optiques (8), et retransmettre ces informations au dispositif distant, pour permettre une surveillance et une commande en temps réel.

9. Construction flottante, comprenant :
le système selon la revendication 7 ou la revendication 8 ;
une unité de traitement configurée pour recevoir et interpréter des instructions provenant du dispositif distant, et les exécuter en envoyant un signal au moyen permettant un positionnement commandable des lentilles optiques (7) et des filtres optiques (8) pour ajuster la position des lentilles optiques (7) et des filtres optiques (8) ;
un système de communication configuré pour permettre à l'unité de traitement de recevoir des données en provenance du dispositif (1), et transmettre ces données à une localisation distante pour un traitement ou une analyse supplémentaire ;
facultativement, une mémoire adaptée pour permettre à l'unité de traitement de stocker des données collectées par le dispositif (1), pour une récupération et une analyse ultérieures.
